(19) Europäisches Patentamt
European Patent Office
Office européen des brevets

(11) Publication number: **0 453 238 A1**

# (12) EUROPEAN PATENT APPLICATION

(21) Application number: **91303383.3**

(51) Int. Cl.$^5$: **A61K 7/075**, A61K 7/50

(22) Date of filing: **16.04.91**

(30) Priority: **20.04.90 GB 9008944**

(43) Date of publication of application:
**23.10.91 Bulletin 91/43**

(84) Designated Contracting States:
**AT BE CH DE DK ES FR GB GR IT LI NL SE**

(71) Applicant: **UNILEVER PLC**
**Unilever House Blackfriars**
**London EC4P 4BQ (GB)**
**GB**
Applicant: **UNILEVER NV**
**Burgemeester s'Jacobplein 1 P.O. Box 760**
**NL-3000 DK Rotterdam (NL)**
**BE CH DE DK ES FR GR IT LI NL SE AT**

(72) Inventor: **Marti, Vernon Peter John**
**Unilever Res. Port Sunlight Lab., Quarry Road East**
**Bebington, Wirral, Merseyside L63 3JW (GB)**
Inventor: **Walsh, Michael Francis**
**Unilever Res. Port Sunlight Lab., Quarry Road East**
**Bebington, Wirral, Merseyside L63 3JW (GB)**

(74) Representative: **Tonge, Robert James et al**
**UNILEVER PLC Patents Division P.O. Box 68**
**Unilever House**
**London EC4P 4BQ (GB)**

## (54) Shampoo composition.

(57) A mild shampoo composition including anionic, amphoteric and alhoxylated or glycosidic nonionic surfactants in a defined weight ratio of 0.9-2.2 :0.5-1.5 :0.9-2.0 respectively and wherein the weight of total surfactants in the shampoo composition is from 8% to 25%.

EP 0 453 238 A1

## Field of the Invention

The present invention relates to shampoo compositions, and more particularly to shampoo compositions which are mild to the hair and scalp.

## Background of the Invention

The mildest shampoos currently available are those marketed specifically as baby shampoos. These are usually based on amphoteric surfactants and they tend not to foam as well as the more conventional shampoo surfactants which are anionic in character.

It is generally the case that increasing the mildness of a detergent composition decreases its foaming power.

This problem was recognised for example in GB 2 165 855 (Colgate-Palmolive) which relates to a nonionic-based light-duty detergent comprising at least 50% by weight of nonionic surfactant, together with anionic surfactant and betaine. Such a formulation, with its high level of nonionic surfactant is unsuitable for use as a shampoo.

It has now been found that a shampoo having improved mildness and adequate foaming may be obtained by careful selection of anionic, amphoteric and nonionic surfactants.

## Brief Summary of the Invention

The invention provides a mild shampoo composition comprising, in addition to water

(a) anionic surfactant;

(b) amphoteric surfactant characterised in that the amphoteric surfactant is not a P-containing betaine compound; and

(c) alkoxylated or glycosidic nonionic surfactant having an HLB of at least 8,

Wherein the weight ratio of (a):(b):(c) lies in the range 0.9 - 2.2:0.5 - 1.5:0.9 - 2.0, and the composition comprises from 8 to 25% by weight of total surfactants (a), (b) and (c).

## DETAILED DESCRIPTION OF THE INVENTION

### Anionic surfactant

The composition of the invention comprises an anionic surfactant which may be selected from alkyl sulphate, alkyl ether sulphate, alkyl sulphonate, alkyl aryl sulphonate, olefin sulphonate, acyl sarcosinate, acyl tauride, acyl isethionate, nonoalkyl sulphosccinate, dialkylsulphosuccinate, acryl lactylate, acylated -amino acid, alkyl carboxylate, monoalkyl phosphate and dialkyl phosphate.

Suitable examples of anionic surfactants which may be included in the compositions of the invention may be selected from but are not necessarily restricted to the following:

alkyl sulphates, such as sodium lauryl sulphate [eg. EMPICOL CX available from Albright & Wilson], and triethanolaminde lauryl sulphate [eg. EMPICOL TL40/T, available from Albright & Wilson];

alkylether sulphates, such as sodium lauryl ether sulphate [eg. EMPICOL ESB70, available from Albright & Wilson];

alkyl sulphonates, such as sodium alkane ($C_{13-18}$) sulphonate [eg. HOSTAPUR SAS 30, available from Hoechst];

alkylaryl sulphonates, such as sodium alkyl benzene sulphonate [eg. TEEPOL CM44, available from Shell];

olefin sulphonates, such as sodium olefin sulphonate ($C_{5-18}$) [eg. HOSTAPUR OS, available from Hoechst];

acyl sarcosinates, having the structural formula:

```
              O
              |
R3  --  C --  N---CH2COOM              (1)
                  |
                 CH3
```

wherein $R^3$ is chosen from $C_{8-14}$ alkyl; and

M is a counterion chosen from alkali metals, ammonium and substituted ammonium such as alkanolammonium.

An example of an acyl sarcosinate having the formula (1), is sodium lauryl sarcosinate [eg. HAMPOSYL L-95, available from Grace].

acyl taurides, having the structural formula:

$$R^4 -- \overset{O}{C} -- \underset{CH_3}{\underset{|}{N}} -- (CH_2)_2SO_3M \qquad (2)$$

wherein $R^4$ is chosen from $C_{8-18}$ alkyl; and M is as defined above for formula (1).

An example of an acyl tauride having the formula (2) is coconut methyl taurine [eg. FENOPEN TC 42, available from GAF];

acyl isethionates, having the structural formula:

$$R^5 -- \overset{O}{C} -- O -- (CH_2)_2SO_3M \qquad (3)$$

wherein $R^5$ is chosen from $C_{8-18}$ alkyl; and M is as defined above for formula (1).

An example of an acyl isethionate having the formula (3) is sodium acyl isethionate [eg. JORDAPON C1, available from Jordon];

monoalkyl suphosuccinates, having the structural formula:

$$R^6 -- O -- \overset{O}{C} -- CH_2\underset{SO_3M}{\underset{|}{CH}} --COOM \qquad (4)$$

wherein $R^6$ is chosen from $C_{10-20}$ alkyl; or $C_{10-20}$ alkyl ethoxy and M is as defined above for formula (1)

Examples of monoalkyl sulphosuccinates having the formula (4) include:

sodium lauryl suphosuccinate [eg EMPICOL SLL, available from Albright & Wilson],

magnesium alkyl sulphosuccinate [eg. ELFANOL 616 Mg, available from AKZO],

sodium lauryl ethoxysulphosuccinate [eg. EMPICOL SDD, available from Albright & Wilson],

coconut monoethanolamide ethoxysulphosuccinate [eg. EMPICOL SGG],

disodium lauryl polyglycolether sulphosuccinate [eg. SURTAGENE S30, available from CHEM-Y], and

polyethyleneglycol sulphosuccinate [eg. REWOPOL SBFA 30, available from REWO],

dialkyl sulphosuccinates, having the structural formula.

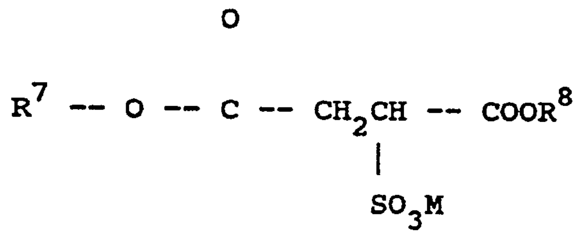 (5)

wherein $R^7$ and $R^8$ are the same or different, and are chosen from $C_{6-14}$ alkyl; and M is defined as above.

Suitable examples of a dialkyl sulphosuccinate having the formula (5) are sodium dilauryl sulphosuccinate [eg. EMCOL 4500, available from Witco], sodium dioctyl sulphosuccinate (eg. Aerosol OT available from Cyanamid]

acyl lactylates, having the structural formula:

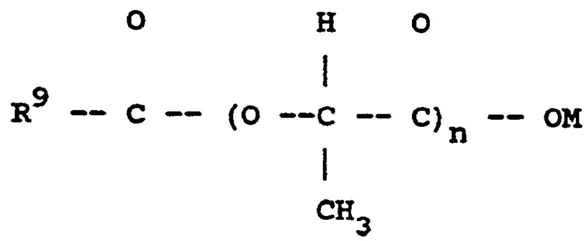 (6)

wherein $R^9$ is chosen from $C_{6-16}$ alkyl; M is as defined above;
and n is 1 or 2.

An example of an acyl lactylate having the formula (6) is decanoyl lactylate [eg. PATIONIC 122A, available from Patterson, CJ];

acylated α -amino acids, such as sodium lauroyl glutamate [eg. ACYLGLUTAMATE LS-11, available from Ajinomoto Co. Inc];

ethyl carboxylates, such as alkyl $C_{12-14}O(EO)_4OCH_2CO_2Na$[eg. AKYPO RLM 38, available from Chem Y]; and

monoalkyl phosphates and dialkyl phosphates, such as dioctyl phosphate.

## Amphoteric surfactant

The shampoo compositions of the invention also comprise amphoteric surfactant. Suitable amphoteric surfactants are derivatives of aliphatic quaternary ammonium, phosphonium and sulphonium compounds, wherein the aliphatic radicals contain from 8 to 18 carbon atoms, and may be straight chain or branched, and further contain an anionic water-solubilising group, such as carboxyl, sulphonate, sulphate, phosphate or phosphonate.

Preferred amphoteric surfactants include -

Alkyl betaines, having the structural formula:

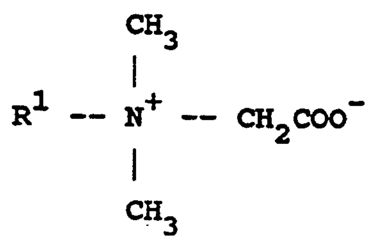 (7)

wherein $R^1$ is $C_{1-16}$ alkyl.

An example of an alkyl betaine having the structure (7) is lauryldimethyl betaine (eg. EMPIGEN BB, available from Albright & Wilson).

Alkylamidopropyl betaines, having the structural formula:

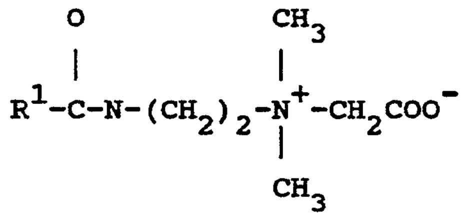

(8)

An example of an alkylamidopropyl betaine having the structure (8) is cocoamidopropyl betaine (eg. TEGOBETAIN L7, available from Goldschmidt);
Alkylamphoglycinates or Alkylamphopropionates having the structural formula:

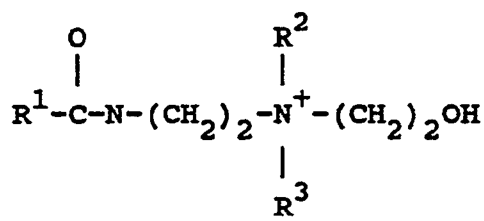

(9)

Wherein $R^2$ is chosen from H,$-CH_2COO^-$ and $-(CH_2)_2COO^-$, and $R^3$ is chosen from $-CH_2COO^-$ and $-(CH_2)_2COO^-$

Suitable examples of compounds (9) are cocoamphoglycinate (available from GAF), and cocoamphopropionate;

Sultaines, having the structural formula;

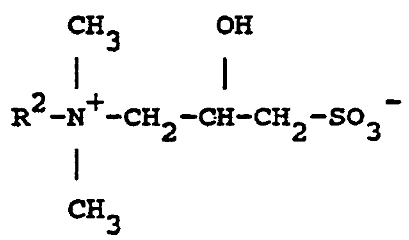

(10)

wherein $R^2$ is chosen from $C_{12-16}$ alkyl alkylamido.

An example of a sultaine having the structural formula (10) is cocoamidopropylhydroxysultaine (eg. CYCLOTERIC BET-CS, available from Alcolac).

The most preferred amphoteric surfactants are lauryl dimethyl betaine and cocoamidopropyl betaine.

Such amphoteric surfactants are considered to contribute to the foaming of the shampoo of the invention, while ameliorating the harshness of the anionic sufactant.

## Nonionic surfactant

The shampoo composition of the invention also comprises alkoxylated or glycosidic nonionic surfactant having an HLB of 8 or more. Above this value nonionics generally form clear isotropic solutions in combination with the other surfactants in the ranges as defined herein. Preferred nonionic surfactants include polyoxyethylene alkyl esters and polyoxyethylene alkyl ethers and alkyl polyglycosides.

A suitable example of a polyoxyethylene alkyl ester, is the CTFA designated Polysorbate 80 which is a mixture of oleate esters of sorbitol and sorbitol anhydrides, condensed with approximately 20 moles of ethylene oxide. Another suitable CTFA designated alkyl ester is Polysorbate 20 which is a mixture of laurate esters of sorbitol and sorbitol anhydrides condensed with approximately 20 moles of ethylene oxide.

Polysorbate 80 and Polysorbate 20 are available commercially as TWEEN 80 and TWEEN 20 respectively, from ICI Americas.

Also suitable for use in the compositions of the invention is the polyethylene glycol ether of $C_{9-11}$ alcohol with an average of 8 ethoxy units, which is available commercially as NONIDET LE-8T (Shell, Japan) or as SYNPERONIC 91-8T (ICI), and the polyethylene glycol ether of $C_{12-15}$ alcohol with an average of 9 ethoxy units

which is available commercially as DOBANOL 25-9 (Shell UK).

Particularly useful alkyl polyglycosides include the glycosides of glucose or glucose oligomers wherein the alkyl chain can be $C_{8-16}$ and the average number of glucose units is 1 to 2. A suitable example is ORAMIX NS 10 (available commercially from SEPPIC) which is the glucoside of $C_{10-12}$ fatty alcohol with an average of about 1.5 glucose units.

The weight ratio of anionic:amphoteric:nonionic surfactants used in the shampoo composition of the invention lies in the range of from 0.9-2.2:0.5-1.5:0.9-2.0, preferably in the range 0.9-2.2:0.9-1.2:0.9-2.0, and the total level of surfactants comprises from 8 to 25% by weight, preferably from 10 to 18% by weight of the composition.

If the composition comprises less than 8% by weight total surfactant there will be inadequate foaming.

We have discovered that by using the three surfactants in the combination of weight ratios set out above, a shampoo is obtained which has improved mildness and satisfactory foaming properties.

The presence of this mildness/foaming combination of weight ratios in the 3-component mixture is quite unexpected, and surprisingly areas outside the combination ratios give low levels of foaming or shampoos which are not sufficiently mild. The exact combination of weight ratios will vary slightly with the particular components used in the composition.

The most important factor in determining the mildness/foaming combination of ratios for any given 3-component mixture appears to be the anionic surfactant. The type of anionic surfactant used has a greater effect on the combination of ratios than either the type of amphoteric or nonionic surfactant used.

Preferred mild shampoos according to the invention may be obtained by using mixtures of anionic surfactant, cocoamidoproyl betaine and $C_{9-11}$ (8EO) primary alcohol ethoxylate. The weight ratio of (a):(b):(c) is determined by the type of anionic surfactant used.

For example, when the anionic surfactant comprises ammonium lauryl sulphate, the mildness/foaming combination of weight ratios is to be found at weight ratio of (a):(b):(c) of 0.9-1.2:0.9-1.2:1.0-2.0.

When the anionic surfactant is sodium lauryl sulphate 2EO, the mildness/foaming combination of weight ratios is to be found at a weight ratio of (a):(b):(c) of 1.2-1.9:0.9-1.2:0.9-1.2.

If sodium lauryl sulphate 3EO is used as the anionic surfactant, the mildness/foaming combination is then to be found at a weight ratio of (a):(b):(c) of 1.3-2.2:0.9-1.2:0.9-1.2

The person skilled in the art will readily ascertain the combination of weight ratios for mildness/foaming when other surfactants are used in the compositions of the invention.

## Other Ingredients

The compositions of the invention may take the form of shampoos. These may also include minor amounts of other ingredients such as antibacterial agents, foam boosters, pearlescers, perfumes, dyes, colouring agents, preservatives, thickeners, proteins, polymers such as silicone polymers, phosphate esters, sunscreens, antidandruff agents and buffering agents. Suitable thickeners include ANTIL 141 (Goldschmidt) which has the CTFA adopted name PEG 55 propylene glycol dioleate and comprises a polyoxyethylene-propylene glycol oleate, and REWOPOL PEG 6000 DS (ex Rewo) which is a polyethylene glycol distearate.

While the invention is particularly concerned with shampoo compositions, it will be apparent to the person skilled in the art that the compositions of the invention, having improved mildness and retaining satisfactory foaming, will also be suitable for other products such as foam bath and shower gel products, or facial wash compositions.

In order to demonstrate the mildness and foaming properties of shampoos of the present invention, the following comparative Example was performed. Shampoos A, B and C according to the invention are compared to a shampoo containing SLES 3EO as the only surfactant and to a commercial baby shampoo, based on nonionic and amphoteric surfactants.

## COMPARATIVE EXAMPLE

### Shampoo A

| | %wt |
|---|---|
| Ammonium lauryl sulphate | 4 |
| Cocoamidopropyl betaine | 4 |
| TWEEN 80 | 4 |
| Water | to 100 |

Shampoo B

| | | %wt |
|---|---|---|
| Sodium lauryl ether sulphate 2EO | | 6 |
| Cocoamidopropyl betaine | | 4 |
| NONIDET LE-8T | | 4 |
| Water | to | 100 |

Shampoo C

| | | %wt |
|---|---|---|
| Sodium lauryl ether sulphate 3EO | | 8 |
| Cocoamidopropyl betaine | | 4 |
| NONIDET LE-8T | | 4 |
| Water | to | 100 |

Assessment of mildness

Mildness was measured by the wheatgerm acid phosphatase technique (WGAP) (C.Prottey, D.Oliver and A.C.Coxon, International Journal of Cosmetic Science, 6, 263,273).

Test solutions, total volume 1.0ml, contained 50mm citrate buffer (pH 4.8), 0.5mm 4-methyl umbelliferyl phosphate (Sigma), and acid phosphatase enzyme (Koch Light) as well as the test shampoo. Solutions were incubated at 37°C for 1 hour. The reaction was terminated by the addition of glycine buffer (0.2M, pH 10.5). The fluorescence of liberated 4-methyl umbelliferone (Sigma) was measured at pH 10.5.

The lower the amount of 4-methyl umbelliferone liberated, the more enzyme inhibition had occurred, thus, the harsher the surfactant system.

Assessment of foaming

The foam generated using shampoos may be assessed by a Foam Machine whose construction and use will now be described.

The Foam Machine

Foam was generated within a glass graduated cylinder by the action of a perforated piston upon the test composition and a suitably treated substrate. The repetitive compression and expansion of the substrate by the piston resulted in the aeration of the composition and the generation of foam which accumulated above the piston plate. The volume of the foam was then read using the graduations of the cylinder when the piston was at its lowest point. The apparatus was enclosed and supplied with air at a constant temperature of 38°C.

The operation of the machine in detail is as follows. Crimped nylon fibre was soaked in a large quantity of a solution of 2.5% by weight artificial sebum, consisting of squalene, cholesterol, long chain fatty acids, long chain triglycerides and decyl oleate, in dichloromethane for 1 hour. The solvent was poured off, and the fibres dried by evaporation of residual solvent. The sebum-treated nylon fibre is intended to simulate greasy human hair to which the composition of the invention would normally be applied when shampooing the hair.

Into a graduated glass cylinder of internal diameter 3.8cm were placed two discs of polyurethane foam (diameter 3.8cm, height 1.1cm). 2g of sebum-treated fibre was placed on top of the foam and the diluted test composition (2 ml) was poured over it. The diluted composition was prepared from 7.5g of test composition and 92.5g of distilled water.

The prepared cylinder was placed within the foam machine, and with the cross-head at its lowest point the

piston plate adjusted to a level of 70 ml (as determined from the graduation on the cylinder).

The machine was operated with a periodicity of 12 rpm. After each minute of operation, the machine was stopped with the cross-head at the highest point and the volume of foam above the piston plate recorded.

The machine was operated for a total of 10 minutes, the reported value being the mean of the foam volume values recorded between 3 and 10 minutes inclusive.

## Results

| Shampoo | WGAP % enzyme inhibition | Foam Volume (ml) |
|---|---|---|
| A | 7.2 | 47.6 |
| B | 0 | 54.9 |
| C | 0 | 54.9 |
| D[1] | 84.0 | 54.0 |
| E[2] | 4.0 | 22.1 |

1. 12% by weight sodium lauryl ether sulphate 3EO
2. Leading baby shampoo containing mainly nonionic and amphoteric surfactants.

It can be seen that shampoos A, B and C are significantly milder than a 12% by weight solution of SLES 3EO, and are at least comparable in mildness to a commercial baby shampoo, shampoos B and C having improved mildness over the baby shampoo.

Foaming of shampoos A, B and C is comparable to a 12% by weight solution of SLES 3EO, and is greatly improved over the baby shampoo.

The invention is further illustrated by the following Examples.

## EXAMPLES

### Example 1

| | %wt |
|---|---|
| Sodium lauryl ether sulphate 3EO | 8.0 |
| Cocoamidopropyl betaine | 4.0 |
| NONIDET LE-8T | 4.0 |
| Polyethylene glycol distearate | 1.0 |
| Perfume, preservative, colour | qs |
| Water | to 100 |

Example 2

| | %wt |
|---|---|
| Ammonium lauryl sulphate | 4.0 |
| Cocoamidopropyl betaine | 4.0 |
| TWEEN 80 | 4.0 |
| Perfume, preservative, colour | qs |
| Water | to 100 |

Example 3

| | %wt |
|---|---|
| Cocoyl polyglycol ether sulphosuccinate | 6.5 |
| Cocoamphoglycinate | 4.8 |
| Nonidet LE-8T | 3.9 |
| Perfume, preservative, colour | qs |
| Water | to 100 |

Example 4

| | %wt |
|---|---|
| $C_{14-16}$ Sodium α-olefin sulphonate | 4.0 |
| Lauryl dimethyl betaine | 3.6 |
| Nonidet LE-8T | 5.0 |
| Perfume, preservative, colour | qs |
| Water | to 100 |

Example 5

| | %wt |
|---|---|
| Cocoyl sarcosinate | 6.3 |
| Cocoamidopropyl hydroxysultaine | 3.0 |
| TWEEN 20 | 6.0 |
| Perfume, preservative, colour | qs |
| Water | to 100 |

Example 6

| | | %wt |
|---|---|---|
| Cocoyl methyl taurate | | 5.0 |
| Lauryl dimethyl betaine | | 3.5 |
| Dobanol 25-9 | | 5.0 |
| Perfume, preservative, colour | | qs |
| Water | to | 100 |

Example 7

| | | wt% |
|---|---|---|
| Sodium lauryl ether sulphate 2EO | | 8.0 |
| Cocoamidopropyl betaine | | 4.5 |
| SYNPERONIC 91-8T | | 4.5 |
| ANTIL 141 | | 1.0 |
| Perfume, preservative, colour | | qs |
| Water | to | 100 |

## Claims

1. A mild shampoo composition comprising, in addition to water
(a) anionic surfactant;
(b) amphoteric surfactant characterised in that the amphoteric surfactant is not a P-containing betaine compound; and
(c) alkoxylated or glycosidic nonionic surfactant having an HLB of at least 8,
wherein the weight ratio of (a):(b):(c) lies in the range 0.9 - 2.2:0.5 - 1.5:0.9 - 2.0, and the composition comprises from 8 to 25% by weight of total surfactants (a), (b) and (c).

2. A shampoo composition as claimed in Claim 1 wherein the weight ratio of (a):(b):(c) lies in the range 0.9-2.2:0.9-1.2:0.9-2.0.

3. A shampoo composition as claimed in Claim 1 or Claim 2 wherein the anionic surfactant is chosen from alkyl sulphate, alkyl ether sulphate, alkyl sulphonate, alkyl aryl sulphonate, olefin sulphonate, acyl sarcosinate, acyl tauride, acyl isethionate, monoalkyl sulphosuccinate, dialkylsulphosuccinate, acyl lactylate, acylated -amino acid, alkyl carboxylate, monoalkyl phosphate and dialkyl phosphate.

4. A shampoo composition as claimed in any one of the preceding claims wherein the anionic surfactant is chosen from sodium lauryl sulphate, ammonium lauryl sulphate, magnesium lauryl sulphate and sodium lauryl ether sulphate 2EO or sodium lauryl ether sulphate 3EO.

5. A shampoo composition as claimed in any one of the preceding claim wherein the amphoteric surfactant is chosen from $C_{10-16}$ alkyl betaines, $C_{10-16}$ alkylamidopropyl betaines, $C_{10-16}$ alkylamphoglycinates,

$C_{10-16}$ alkyl amphopropionates and $C_{12-28}$ alkyl sultaines.

**6.** A shampoo composition as claimed in Claim 5 wherein the amphoteric surfactant is chosen from lauryl dimethyl betaine and cocoamidopropyl betaine.

**7.** A shampoo composition as claimed in any one of the preceding claims wherein the nonionic surfactant is chosen from the group polyoxyethylene alkyl ester and polyoxyethylene alkyl ether.

**8.** A shampoo composition as claimed in Claim 7 wherein the nonionic surfactant is chosen from polysorbate 80, polysorbate 20, polyethylene glycol ether of $C_{9-11}$ alcohol with an average of 8 ethoxy units, polyethylene glycol ether of $C_{12-15}$ alcohol with an average of 9 ethoxy units and a glucoside of $C_{10-12}$ fatty alcohol with an average of about 1.5 glucose units.

**9.** A shampoo composition as claimed in any one of the preceding claims wherein the composition comprises ammonium lauryl sulphate, cocoamidopropyl betaine and nonionic surfactant in the weight ratio 0.9 - 1.2:0.9 - 1.2:0.9 - 2.0.

**10.** A shampoo composition as claimed in Claims 1 to 8 wherein the composition comprises sodium lauryl sulphate 2EO, cocoamidopropyl betaine and the nonionic surfactant in the weight ratio 1.2 - 1.9:0.9 - 1.2:0.9 - 1.2.

**11.** A shampoo composition as claimed in Claim 10 wherein the amount of sodium lauryl sulphate 2EO is 8% by weight and the amount of cocoamidopropyl betaine is 4% by weight of the total composition.

**12.** A shampoo composition as claimed in any one of Claims 1 to 8 wherein the composition comprises sodium lauryl sulphate 3EO, cocoamidopropyl betaine and the nonionic surfactant in the weight ratio 1.3 - 2.2:0.9-1.2:0.9 - 1.2.

**13.** A shampoo composition as claimed in any one of the preceding claims wherein the composition comprises from 10 to 18% by weight of total surfactants (a), (b) and (c).

**14.** A shampoo composition as claimed in any one of the preceding claims further comprising an organic thickening agent.

**15.** A shampoo composition as claimed in Claim 14 wherein the organic thickening agent is selected from polyoxyethylene-propylene glycol dioleate and polyethylene glycol distearate.

European Patent Office

# EUROPEAN SEARCH REPORT

Application Number

EP 91303383.3

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl.5) |
| X | US - A - 3 950 417 (R.J. VERDICCHIO et al.) * Abstract; column 2, line 54 - column 6, line 27 * | 1-5,13 | A 61 K 7/075<br>A 61 K 7/50 |
| X | EP - A2 - 0 127 580 (CIBA-GEIGY AG) * Page 10, line 18 - page 31, line 17 * | 1-8, 13-15 | |
| A | US - A - 4 329 334 (D.T. SU et al.) * Claims; column 4, line 36 - column 6, line 11; column 7, lines 1-21 * | 1-15 | |
| A | H. JANISTYN "Handbuch der Kosmetika und Riechstoffe", second edition, vol. III, Die Körperpflegemittel, 1973, Dr. Alfred Hüthig Verlag Heidelberg pages 228-254 * Pages 229-234,239,240,247, lines 6-9 * | 1-15 | TECHNICAL FIELDS SEARCHED (Int. Cl.5)<br>A 61 K 7/00 |

The present search report has been drawn up for all claims

| Place of search | Date of completion of the search | Examiner |
|---|---|---|
| VIENNA | 25-06-1991 | IRMLER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)